(19) Europäisches Patentamt
European Patent Office
Office européen des brevets

(11) **EP 3 436 055 B1**

(12) **EUROPEAN PATENT SPECIFICATION**

(45) Date of publication and mention
of the grant of the patent:
**25.10.2023 Bulletin 2023/43**

(21) Application number: **17719952.8**

(22) Date of filing: **27.03.2017**

(51) International Patent Classification (IPC):
**A61K 38/54** (2006.01)       **A61L 2/08** (2006.01)
**C12N 9/94** (2006.01)       **A61P 1/18** (2006.01)

(52) Cooperative Patent Classification (CPC):
**A61K 38/465; A61K 38/54; A61L 2/08; A61P 1/18;
C12N 9/20; C12N 9/94; C12Y 301/01003**

(86) International application number:
**PCT/US2017/024315**

(87) International publication number:
**WO 2017/172619 (05.10.2017 Gazette 2017/40)**

(54) **ENZYME COMPOSITIONS WITH REDUCED VIRAL AND MICROBIAL CONTAMINATION**

ENZYMZUSAMMENSETZUNGEN MIT REDUZIERTER VIRALER UND MIKROBIELLER KONTAMINATION

COMPOSITIONS D'ENZYME À CONTAMINATION MICROBIENNE ET VIRALE RÉDUITE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **28.03.2016 US 201662314048 P**
**31.01.2017 US 201762452746 P**
**03.02.2017 US 201762454184 P**

(43) Date of publication of application:
**06.02.2019 Bulletin 2019/06**

(73) Proprietors:
• **Abbott Laboratories GmbH**
**30173 Hannover (DE)**
• **Abbott GmbH**
**65205 Wiesbaden (DE)**

(72) Inventors:
• **BABCOCK, Martin**
**Pleasant Prairei, WI 53158 (US)**
• **BURNELL, Cynthia**
**Gurnee, IL 60031 (US)**
• **KALTHOD, Vikram**
**Mattawan, MI 49071 (US)**
• **BREITENBACH, Jörg**
**Wiesbaden, 65205 (DE)**
• **SCZESNY, Frithjof**
**Hannover, 30173 (DE)**
• **SHLIEOUT, George**
**Hannover, 30173 (DE)**
• **RUEFFER, Frauke-Regina**
**Hannover, 30173 (DE)**
• **HESS, Mark**
**Palatine, Illinois 60067 (US)**
• **DETE, John**
**Graylake, Illinois 60030 (US)**
• **CRANDALL, Daniel**
**La Grange Park, Illinois 60526 (US)**
• **HERTZLER, Shannon**
**Green Oaks, Illinois 60048 (US)**
• **RIORDAN, William**
**Libertyville, Illinois 60048 (US)**
• **SANDERS, Houston**
**Kenosha, Wisconsin 53144 (US)**

(74) Representative: **Vossius & Partner
Patentanwälte Rechtsanwälte mbB
Siebertstrasse 3
81675 München (DE)**

(56) References cited:
**WO-A2-2015/019198      US-A1- 2006 011 376
US-A1- 2010 119 654**

- **O Ferdes ET AL: "IAEA-SM-350/25 THE USE OF DIFFERENT TYPE OF ELECTRON BEAM RADIATION EQUIPMENT FOR BIOTECHNOLOGICAL MATERIALS", , 1 January 1998 (1998-01-01), XP055379497, Retrieved from the Internet: URL:http://www.iaea.org/inis/collection/NC LCollectionStore/_Public/29/050/29050436.p df?r=1 [retrieved on 2017-06-08]**

Remarks:

The file contains technical information submitted after the application was filed and not included in this specification

**Description**

**TECHNICAL FIELD**

The present invention relates to pancreatin enzyme preparationproduced by a method of subjecting intact mammalian pancreatic tissue to electron beam radiation and isolating pancreatin from the irradiated pancreatic tissue, methods of production of such preparations and.the use of the preparations in the treatment of exocrine pancreatic insufficiency.

**BACKGROUND**

Products derived from animal tissue may exhibit viral and/or microbial contamination. Certain biological contaminants such as bacteria or protozoa may be inactivated during manufacturing processes. However, other biological contaminants such as non-enveloped viruses are resistant to established methods for reduction or inactivation of contaminants. A particular challenge is the inactivation or removal of viruses from enzyme compositions derived from animal tissue without destroying or changing the activity of the enzymes in the process.

Established methods for viral inactivation include, for example, pasteurization, dry heat, vapor heat, solvent/detergent treatment, and low pH. The selection of the methods to be employed for viral inactivation depend on the nature and contamination of the product, the method of purification used, if any, and the nature of the viral contaminant. For example, solvent or detergent treatment can disrupt the lipid membrane of enveloped viruses and has thus been used for inactivation of enveloped viruses. However, many non-enveloped viruses are generally not inactivated by solvent or detergent treatment.

Heat, in particular dry heat, is another established method for viral inactivation. While dry heat treatment may inactivate even highly resistant viruses, such as non-enveloped viruses, the treatment requires extended time periods (of several hours) and monitoring of moisture content. Moreover, heat treatment can compromise the desired biological activity of the product, particularly where the product is an enzyme composition.

Pancreatic enzyme products have long been used to treat exocrine pancreatic insufficiency, a condition associated with cystic fibrosis (CF), chronic pancreatitis, obstruction of the pancreas or common bile duct (such as from a neoplastic disease), surgical procedures such as pancreatectomy or gastrointestinal bypass surgery, as well as other diseases and disorders. The pancreas secretes digestive enzymes, including lipases, proteases, and amylases, into the proximal duodenal lumen, where they facilitate the hydrolysis of macronutrients. Amylases and proteases are secreted by organs other than the pancreas, and these contribute to the digestion of carbohydrates and protein. However, there is relatively little lipase from sources other than the pancreas involved in digestion of lipids. Thus, patients with untreated exocrine pancreatic insufficiency typically have difficulty digesting fat and may suffer symptoms of maldigestion or malnutrition or both, with deficiencies of essential fatty acids and fat-soluble vitamins, weight loss, cramping, flatulence, bloating, and greasy, foul-smelling, loose stools (steatorrhea). For patients with CF, inadequate treatment may have serious consequences, as good nutritional status has been directly correlated with good lung function.

Pancreatic enzyme therapy treats and/or avoids malabsorption and facilitates growth and development in patients with exocrine pancreatic insufficiency. In patients with CF, mucus blocks the pancreatic duct in the pancreas as it does in the lungs. The pancreatic digestive enzymes are not secreted into the intestine, and thereby digestion of starch, fat, and protein is impaired. The lack of digestion results in steatorrhea, abdominal pain, and weight loss, among others.

Maldigestion in mammals and humans is usually based on a deficiency of digestive enzymes, in particular on a deficiency of endogenous lipase, but also of protease and/or amylase. If the pancreatic insufficiency is pathological, this may be congenital or acquired. Acquired chronic pancreatic insufficiency may, for example, be ascribed to alcoholism. Congenital pancreatic insufficiency may, for example, be ascribed to the congenital disease cystic fibrosis. The consequences of the deficiency of digestive enzymes may be severe symptoms of under-nutrition and malnutrition, which may be accompanied by increased susceptibility to secondary illnesses.

Substitution with exogenous digestive enzymes or mixtures of digestive enzymes (i.e., pancreatic enzyme therapy) has proved an effective treatment for a deficiency in endogenous digestive enzymes. Most frequently, pharmaceutical preparations containing porcine pancreatin are used for pancreatic enzyme therapy (also known as "enzyme substitution therapy"). For such pharmaceutical preparations, the active ingredient evaluated in clinical trials is lipase and dosage amounts for commercial products are given in lipase units. Nevertheless, such mixtures of digestive enzymes obtained from the pig pancreas comprise lipases, amylases and proteases, and can be used effectively for pancreatic enzyme therapy in humans owing to the great similarity of the enzymes and accompanying substances contained therein to the contents of human pancreatic juices. The pancreatic enzymes are usually administered orally in the form of solid preparations.

Pancreas glands may be obtained from animals, such as pigs, raised and slaughtered for food. Governmental regulations often require that pancreas glands be obtained from a single species slaughterhouse (i.e., no other species are slaughtered and processed at that facility) and, thereby limit the availability of starting material. Wide-spread contamination of facilities with infectious agents may lead to a shutdown of production and to supply shortfall. Current testing procedures may identify contaminated lots and elimination of such lots place further burdens on an already constrained supply of starting material.

Processes to obtain pancreatic enzyme(s) from a mammalian pancreas gland are available. For example, processes are described in US 4,623,624 by which pancreatin is obtained through autolysis of an aqueous isopropanol-containing tissue slurry.

The presence of infectious agents, and in particular viruses, in porcine pancreas used for manufacture of pancreatin is recognized. Indeed, most swine herds have been infected with porcine parvovirus (PPV), which has a high resistance to inactivation. PPV has been detected in pancreatin as an infectious agent. Although, PPV is not believed to be pathogenic for humans, it is desirable to obtain pancreatin with a reduced PPV load. In addition, PPV is a common model virus as it is difficult to inactivate by standard methods, such as chemical or thermal processing.

US 2010/0119654 relates to irradiation of an alcoholic or aqueous biological extract which contains solids in the form of a suspension. The radiation employed in US 2010/0119654 is ultra-violet (UV) radiation, x-ray radiation, $\beta$ radiation, or $\gamma$-radiation. UV irradiation of a pancreatin intermediate dissolved in 40% isopropanol produced up to 4 $\log_{10}$ reduction in M2 phage. Gamma-irradiation of pancreatin (API) produced an approx. 40% decrease in lipase activity at 27 kGy and a 13% decrease in lipase activity at 5 kGy. Bacterial content was reduced by more than 2.5 $\log_{10}$ but virus inactivation was not reported. When pancreatin (API) was treated with $\beta$-irradiation, >85% enzyme activity was reportedly maintained, but "germ count" was only reduced by approx. 1.5 $\log_{10}$.

WO 2003/020324 relates to sterilizing digestive enzymes, such as trypsin, $\alpha$-galactosidase, and iduronate 2-sulfatase, with irradiation. Lyophilized or liquid enzymes (trypsin, a glycosidase, or a sulfatase) were irradiated alone or in the presence of a stabilizer. $\gamma$-irradiation was accomplished using a $^{60}$Co source. Viral inactivation was not reported.

WO 2007/014896 relates to reducing the concentration of one or more biological, in particular viral, contaminants of pancreatin by heating the pancreatin.

In US 2009/0233344, heat treatment of pancreatin at 80°C for 32 hours provided about 2.5 $\log_{10}$ reduction in PPV viral titer but also a 20% loss in lipase activity. Heat treatment of pancreatin at 100°C for 8 hours provided a greater than 3 $\log_{10}$ reduction in PPV viral titer, but nearly 50% of lipase activity was lost.

Thus, process steps that can be effective against difficult-to-inactivate viruses, such as PPV, have a high potential for changing the nature of the pancreatin product by degrading or reducing the pancreatic enzymes, particularly lipase, to unacceptable levels. Such changes in potency may reduce or alter the efficacy profile of the ultimate product. Therefore, it is desirable to maintain enzyme activity, particularly lipase activity, during the manufacturing process.

Since each of the previously tested viral clearance processes that demonstrated some effectiveness against difficult-to-inactivate viruses (*e.g.*, PPV) resulted in significant loss of enzyme activity, including lipase activity, there was skepticism in the industry that a robust level of viral inactivation/clearance could be achieved without compromising product quality. In particular, there was skepticism in the industry that a suitable robust, orthogonal viral clearance step could be developed without adversely impacting the chemical, physical, or pharmaceutical properties of pancreatin. *See, e.g.,* Letter from Scientific Protein Laboratories to FDA dated June 22, 2004 in Docket No. 2003D-0206.

## SUMMARY OF THE INVENTION

The invention is set out in the appended set of claims.

The present disclosure pertains to pancreatin enzyme preparations (i.e. pancreatin or pancreatin enzyme preparation) that may be isolated from an animal tissue source that include one or more enzymes, that have a reduced viral and/or microbial contamination relative to the source animal tissue, and maintain at least one biological activity of the source animal tissue. The enzyme preparation is produced by subjecting the source animal tissue to electron beam radiation, and subsequently isolating the pancreatin from the irradiated tissue. The source animal tissue is intact tissue. The irradiated tissue exhibit at least a three $\log_{10}$, preferably at least a four $\log_{10}$, reduction in viral load of a model virus compared to a control sampleOrthogonal viral reduction steps may be employed (e.g., during the step of isolating one or more enzymes from the irradiated tissue). In certain embodiments, the enzyme preparation isolated from the irradiated tissue has a biological activity corresponding to at least 50%, preferably at least 90%, of the biological activity of a control enzyme preparation, such as an enzyme preparation isolated from non-irradiated source animal tissue. In certain embodiments, the biological activity is lipase activity. The irradiated tissue may exhibit at least a three $\log_{10}$, preferably at least a four $\log_{10}$, reduction in viral and/or microbial contaminants compared to non-irradiated source animal tissue and the enzyme preparation isolated from the irradiated tissue may have a biological activity corresponding to at least 50%, preferably at least 90%, of the biological activity of a control enzyme preparation, such as an enzyme preparation isolated from non-irradiated source animal tissue.

A method for producing a pancreatin enzyme preparation derived from intact mammalian pancreatic tissue, wherein the method includes a treatment sufficient to produce at least a three $\log_{10}$, preferably at least a four $\log_{10}$, reduction in viral and/or microbial contaminants compared to the source animal tissue. The treatment of the invention comprises subjecting intact source mammalian pancreatic tissue to electron beam radiation, to produce irradiated pancreatic tissue. The electron beam radiation treatment may be sufficient to reduce viral and/or microbial contamination of the source animal tissue, while maintaining at least one biological activity of the source animal tissue. In certain embodiments, the biological activity is lipase activity. One or more enzymes and/or proenzymes may be extracted from the irradiated animal tissue. One or more enzymes are isolated from the irradiated pancreatic animal tissue. The method may reduce the risk of infectious contamination of the animal-derived enzyme preparation or a pharmaceutical composition comprising the animal-derived enzyme preparation relative to an untreated control.

Another aspect of the present invention pertains to a pharmaceutical composition comprising the enzyme preparations described herein, wherein the enzyme preparation comprises or is pancreatin. The pharmaceutical composition may be an oral pharmaceutical dosage form. The pharmaceutical composition may be used in the treatment or prevention of a disease responsive to pancreatic enzyme replacement therapy, such as exocrine pancreatic insufficiency. Thus, another aspect of the present invention pertains to pancreatin enzyme preparation or pharmaceutical composition described herein for use in the treatment or prevention of exocrine pancreatic insufficiency.

## DETAILED DESCRIPTION OF THE INVENTION

### A. DEFINITIONS

As used in the specification and the appended claims, unless specified to the contrary, the following terms have the meaning indicated:

The term "API" as used herein stands for "active pharmaceutical ingredient." The preferred API as disclosed herein is pancreatin, in particular porcine pancreatin as generally used for therapeutic purposes, *i.e.,* pancreatin according to the requirements of standard pharmacopoeias, e.g. Ph. Eur. and/or USP and suitable for oral administration in the treatment or prophylaxis of maldigestion in mammals, in particular humans, and in particular of maldigestion due to chronic exocrine pancreatic insufficiency such as in patients suffering from cystic fibrosis, chronic pancreatitis or patients who have undergone upper gastrointestinal surgery.

The term "crude" as used herein refers to a non-purified preparation or mixture containing enzymes and/or proenzymes as well as additional components derived from the source tissue. A crude preparation or mixture includes, but is not limited to, animal tissue itself.

The term "enzyme preparation" refers to any composition of matter containing one or more enzymes, whether in the active or inactive form (*i.e.*, proenzymes or zymogens). The term includes cell or tissue extracts as well as crude preparations derived from animal tissue or other cellular material. One example of an enzyme preparation is pancreatin, pancrelipase, an extract derived from mammalian, preferably porcine, pancreatic The enzymatic preparation of the invention refers to pancreatin enzymatic preparation, i.e. pancreatin.

The term "extract" as it relates to enzyme preparations as used herein refers to one or more enzymes and/or proenzymes that have been separated from at least one component of the tissue from which they were derived. Extracted components may be in the form of an active enzyme or a proenzyme (zymogen) requiring subsequent conversion to the active form.

The term "isolate" as it relates to enzyme preparations as used herein refers to one or more active enzymes that have been separated from at least one component of the tissue from which they were derived. Thus, in certain embodiments, an "isolated enzyme" or an "isolated enzyme preparation" includes one or more active enzymes that have been converted from the corresponding proenzyme form via hydrolysis and/or autolysis. Hydrolysis and/or autolysis to convert the proenzyme to an active enzyme may occur before, during, or after extraction.

The terms "pancreatic enzymes", "pancreatin" and "pancrelipase" as used herein refer to enzymatic mixtures derived from mammalian pancreatic glands comprising digestive enzymes such as lipase, protease and amylase as main components. In particular, the terms "pancreatic enzymes", "pancreatin" and "pancrelipase" may be used synonymously herein and refer to pancreatic extracts suitable for therapeutic use, in accordance with standard pharmacopoeias, which contain several digestive enzymes whose properties are defined by standard monographs as explained above. Due to standard manufacturing processes, "pancreatic enzymes", "pancreatin" and "pancrelipase" are usually provided in powder form as "pancreatin powder", sometimes also referred to as "pancreas powder". Pancreatic enzymes, pancreatin and pancrelipase also can be, and preferably are, APIs. Pancreatin for pharmaceutical use is typically of bovine or porcine origin. Porcine pancreatin is preferred. Pancrelipase has been described in some references as an enzyme preparation with increased activity (lipase) relative to pancreatin.

As used herein, the term "pharmaceutical composition" means a composition comprising an enzyme preparation as described herein and optionally one or more pharmaceutically acceptable excipients.

The term "pharmaceutically acceptable" is used adjectivally to mean that the modified noun is appropriate for use as a pharmaceutical product or as a part of a pharmaceutical product.

An "orthogonal" microbial and/or viral reduction step refers to a distinct method for reduction of the microbes and/or viruses that may be present in a sample. A microbial and/or viral reduction step can be orthogonal provided that there are one or more additional microbial and/or viral reduction steps in the process. In certain embodiments, an "orthogonal" microbial and/or viral reduction step has a sufficiently distinct mechanism from all other microbial and/or viral reduction steps used in the process such that the $\log_{10}$ kill achieved by the "orthogonal" step becomes additive with the cumulative $\log_{10}$ kill achieved from the all other microbial and/or viral reduction steps that are used for obtaining the enzyme preparation.

The terms "prevent", "preventing" and "prevention" refer to a method of preventing the onset of a condition, disorder, or disease and/or the attendant symptoms thereof or barring a subject from acquiring a condition, disorder, or disease. As used herein, "prevent", "preventing" and "prevention" also include delaying the onset of a condition, disorder, or disease and/or the attendant symptoms thereof and reducing a subject's risk of acquiring a condition, disorder, or disease.

The term "subject" includes humans and other primates as well as domesticated and semi-domesticated animals including, but not limited to, poultry, honeybees, cows, sheep, goats, pigs, horses, dogs, cats, rabbits, rats, mice and the like. The term "poultry" encompasses all types of domestic fowl, including, but not limited to chickens, turkey, ducks, geese, the ratite group of birds and game birds. The subject may be a human

The term "therapeutically effective amount" means a sufficient amount of the enzyme preparation or pharmaceutical composition for use in the treatment of a condition, disorder, or disease, at a reasonable benefit/risk ratio applicable to any medical treatment. When used in a medical treatment, a therapeutically effective amount of one of the enzyme preparations can be employed as an extract or in a crude form. Alternatively, the enzyme composition can be administered as a pharmaceutical composition containing the enzyme composition of interest in combination with one or more pharmaceutically acceptable carriers.

The terms "treat", "treating" and "treatment" refer to a method of alleviating or abrogating a condition, disorder, or disease and/or the attendant symptoms thereof.

## B. ENZYME PREPARATIONS AND METHODS OF MANUFACTURE

Disclosed herein is a pancreatin enzyme preparation i.e. pancreatin. The enzyme preparation may comprise a proenzyme, such as a prolipase or a typsinogen. The enzyme preparation may be in a crude form. The enzyme preparation comprises one or more proenzymes that have been extracted from intact mammalian pancreatic tissue. The enzyme preparation has been isolated from an intact mammalian pancreatic tissue.

An isolated enzyme preparation may have the same or substantially the same biological activity, but less infectiousness, as the tissue from which it was isolated. The isolated enzyme preparation may have the same or substantially the same biological activity as a control enzyme preparation. The infectiousness of the isolated enzyme preparation may be reduced by at least three $\log_{10}$, preferably at least four $\log_{10}$, relative to the infectiousness of the tissue from which it was isolated. The infectiousness that is reduced is viral infectiousness, particularly, non-enveloped viral infectiousness and/or enveloped virus infectiousness. A biological activity of the isolated enzyme preparation may be at least 50%, at least 60%, at least 70%, at least 80%, or preferably at least 90%, of the biological activity of a control enzyme preparation. The biological activity may be enzymatic activity, such as protease activity, amylase activity, or, preferably, lipase activity.

The pancreatin enzyme preparation is isolated from a pre-treated pancreatic tissue source and may have the same or substantially the same biological activity, but less infectiousness, than a control preparation isolated from an untreated tissue source. The infectiousness of the enzyme preparation may be reduced by at least a three $\log_{10}$, preferably at least a four $\log_{10}$, relative to the control preparation. The infectiousness of the pre-treated tissue source may be reduced by at least a three $\log_{10}$, preferably at least a four $\log_{10}$, relative to the untreated tissue source. The infectiousness that is reduced is viral infectiousness, particularly, non-enveloped viral infectiousness. A biological activity of the enzyme preparation may be at least 50%, at least 60%, at least 70%, at least 80%, or preferably at least 90%, of the biological activity of a control preparation isolated from an untreated tissue source. The biological activity may be enzymatic activity, such as protease activity, amylase activity, or, preferably, lipase activity.

The pancreatin enzyme preparation is derived from an electron beam irradiated intact mammalian pancreatic tissue. The electron beam irradiated tissue may be porcine pancreatic tissue. The enzyme preparation derived from electron beam irradiated tissue comprises pancreatin. The enzyme preparation isolated from electron beam irradiated pancreatic tissue may comprise a proenzyme, such as a prolipase or a typsinogen. The enzyme preparation isolated from electron beam irradiated pancreatic tissue may be in a crude form. The enzyme preparation isolated from electron beam irradiated pancreatic tissue may comprise one or more enzymes and/or proenzymes that have been extracted from the irradiated tissue.

The pancreatin enzyme preparation is isolated from an electron beam irradiated intact mammalian pancreatic tissue and may have the same or substantially the same biological activity, but less infectiousness, than a control preparation isolated from a non-irradiated tissue source. The irradiated tissue is intact mammalian pancreatic tissue. In certain embodiments, the irradiated tissue is flaked pancreatic tissue, a whole pancreas gland, or a portion of a whole pancreas gland. The non-irradiated tissue source is mammalian pancreatic tissue. The infectiousness of the enzyme preparation may be reduced by at least a three $\log_{10}$, preferably at least a four $\log_{10}$, relative to the control preparation. The infectiousness that is reduced may be viral infectiousness, particularly, non-enveloped and/or enveloped viral infectiousness. In certain embodiments, the infectiousness that is reduced may be PPV infectiousness. A biological activity of the isolated enzyme preparation may be at least 50%, at least 60%, at least 70%, at least 80%, or preferably at least 90%, of the biological activity of the control preparation. The biological activity may be enzymatic activity, such as protease activity, amylase activity, or, preferably, lipase activity.

The enzyme preparation may comprise electron beam irradiated proenzymes. The enzyme preparation may be further processed, such as by converting the irradiated proenzymes into their active form (*e.g.*, by autolysis and/or hydrolysis).

The present pancreatin enzyme preparations can be better understood in connection with the following methods which illustrate exemplary techniques by which the enzyme preparations can be obtained.

The present disclosure includes a method for manufacturing a pancreatin product. The method comprises subjecting intact mammalian pancreatic tissue to electron beam radiation. The intact mammalian pancreatic tissue may be a frozen tissue block. In certain embodiments, the intact mammalian pancreatic tissue is flaked or minced animal tissue.

The method begins with intact mammalian pancreatic tissue. The mammalian pancreatic tissue may be porcine pancreas. Porcine pancreas may be procured from an approved slaughterhouse, preferably a single species slaughterhouse.

Intact pancreatic tissue includes a whole pancreas gland or a portion thereof, such as one or more lobes. Intact pancreatic tissue may include flaked frozen tissue. Intact pancreatic tissue may include a frozen tissue block, which may have been mechanically processed. Intact pancreatic tissue may include pancreatic tissue that has been minimally manipulated or altered, or preferably not manipulated or altered, in such a way as to, for example, destroy active enzymes and/or convert proenzymes in the tissue to their active form. For example, a tissue homogenate that undergone significant chemical or enzymatic processing to convert proenzymes to their active form is not "intact tissue" as the term is used herein. As another example, tissue that has been ground or minced under conditions that destroy active enzymes and/or convert proenzymes in the tissue to their active form is not "intact tissue" as the term is used herein.

The intact mammalian pancreatic tissue, which may be frozen animal tissue, may be comminuted. Comminution can be achieved using a frozen block flaker, such as a Hydrauflake Chunker provided by General Machinery Corporation (Sheboygan, WI), which is designed to chunk frozen tissue in preparation for further processing.

The intact mammalian pancreatic tissue is irradiatedby exposure to a sterilizing beam of accelerated electrons, i.e., an E-beam or electron beam radiation. In certain embodiments, a whole pancreas gland or an intact portion thereof is exposed to electron beam irradiation. Flaked porcine pancreatic tissue may be exposed to electron beam irradiation.

E-beam radiation is a form of ionizing energy that is generally characterized by its low penetration and high dosage rates. The beam, a concentrated, highly charged stream of electrons, is generated by the acceleration and conversion of electricity. The electrons are generated by equipment referred to as accelerators, which are capable of producing beams that are either pulsed or continuous. As the material being irradiated passes beneath or in front of the electron beam, energy from the electrons is absorbed. This absorption of energy alters various chemical bonds and biological properties within the product/material. The energy that is absorbed is referred to as the "absorbed dose." It is this absorption of energy--or "dose delivery"--that destroys viruses and microorganisms, e.g., by destroying their DNA or RNA chains.

Irradiation may be carried out in a conventional manner, such as by placing the tissue in a suitable container and exposing the tissue to an electron beam. The container holding the tissue may be placed on a conveyor which then passes through the electron beam. The container holding the tissue may not contain any solvent. The container holding the tissue may be substantially free of solvent. The container holding the tissue may not contain any flammable solvent. The container holding the tissue may be substantially free of a flammable solvent, such as alcohol. For example, the container may contain frozen, intact tissue such as a whole gland, a portion of a whole gland, or flaked tissue.

The radiation is provided at a dose sufficient to produce at least a three $\log_{10}$ reduction in viral load of a model virus compared to a control sample. The radiation may be at a dose that prevents loss of a biological activity, preferably an enzymatic activity, relative to a control enzyme preparation isolated from non-irradiated tissue.

The beam of accelerated electrons may be provided by an electron accelerator, such as an electron accelerator provided by Iotron Industries USA, Inc. (Columbia City, IN). In certain embodiments, the electron accelerator operates at powers from 20 to 250 kW and beam energies from 5 to 18 mega electron-volts (MeV). The electron accelerator may operate at 60 kW and 10 MeV. The electron accelerator may provide a beam energy of 10 MeV or above.

A tissue can be exposed to electron beam irradiation for a time and in an amount sufficient to achieve viral or microbial inactivation without compromising a biological activity of one or more enzymes subsequently extracted or isolated from the irradiated tissue. The dosage of electron beam irradiation required to sterilize a tissue can vary based on, for example, the size of the tissue, the type of the tissue, and the type and amount of viral or microbial contaminant in, or suspected of being present in, the tissue sample. One skilled in the art will recognize and be able to determine an appropriate dose and time suitable for a particular tissue and based on the characteristics of the tissue and accelerator being used. The electron beam dose selected may be effective for inactivation of infectious agents that are difficult to destroy by conventional processes while causing minimal loss in enzyme activity.

An "absorbed dose" of radiation is expressed in terms of kilograys (kGy), wherein one kilogray is equal to one thousand joules of energy deposited per kilogram of material. The tissue is exposed to the electron beam until an infectious agent-inactivating amount of radiation is absorbed. For example, the tissue may be exposed to the electron beam until a dose of about 10, about 15, about 20, about 25, about 30, about 35, about 40, about 45, or about 50 kilograys (kGy) or more is achieved. As another example, the tissue may be exposed to the electron beam until a dose of about 5 to about 50, about 10 to about 40, about 15 to about 35 kGy is achieved. The tissue may be exposed to the electron beam until a dose of about 30 kGy is absorbed. Porcine pancreas may be irradiated with an electron beam dose sufficient to provide a high $\log_{10}$ kill for infectious agents, preferably infectious agents that are difficult to inactivate such as non-enveloped viruses, while causing minimal loss in enzyme activity.

Dosage can be determined with the use of radiochromic dye films. Such films can be calibrated by reference to a national standard.

The tissue is exposed to electron beam radiation for a time and in an amount sufficient to produce at least a three $\log_{10}$, preferably at least a four $\log_{10}$, reduction in viral load of a model virus compared to a control sample. The tissue may be exposed to electron beam radiation for a time and in an amount sufficient to produce between about three $\log_{10}$ and about five $\log_{10}$ reduction in viral load of a model virus compared to a control sample. The tissue may be exposed to electron beam radiation for a time and in an amount sufficient to produce about four $\log_{10}$ reduction in viral load of a model virus compared to a control sample. In certain embodiments, the model virus is PPV. The tissue is intact mammalian pancreatic tissue. In some such embodiments, the intact tissue is a a whole pancreas gland or a portion thereof, such as one or more lobes of a pancreas gland. The intact tissue may be frozen, flaked tissue.

The electron beam exposure may include single-sided or multiple-sided exposure. The tissue may be subjected to a one-sided exposure to the electron beam. The tissue may be subjected to a multiple-sided exposure, for example, a two-sided exposure to the electron beam. Thus, a dose of about 20 kGy may comprise a two-sided exposure at about 10 kGy/side; a dose of about 30 kGy may comprise a two-sided exposure at about 15 kGy/side; a dose of about 40 kGy may comprise a two-sided exposure at about 20 kGy/side; and a dose of about 50 kGy may comprise a two-sided exposure at about 25 kGy/side.

The methods of manufacturing the pancreatin enzyme preparation may reduce the risk of viral or microbial infectiousness of a pharmaceutical composition comprising the enzyme preparation.

Infectiousness of an irradiated pancreas gland may be reduced by at least a three $\log_{10}$, preferably at least a four $\log_{10}$, relative to the infectiousness of the gland prior to radiation treatment. Infectiousness of a pancreatin enzyme preparation derived or isolated from an irradiated pancreas gland may be reduced by at least a three $\log_{10}$, preferably at least a four $\log_{10}$, relative to the infectiousness of the gland prior to radiation treatment. Alternatively, the infectiousness of the pancreatin enzyme preparation may be determined relative to a control enzyme preparation derived or isolated from a non-irradiated pancreas gland.

The infectiousness that is reduced may be viral infectiousness, particularly, the viral infectiousness of a non-enveloped virus such as PPV. For example, PPV infectiousness of a pancreatin product isolated from an irradiated pancreas gland may be reduced by at least a three $\log_{10}$, preferably at least a four $\log_{10}$ relative to a control enzyme preparation isolated from a non-irradiated pancreas gland. As another example, PPV infectiousness of an irradiated pancreas gland may be reduced by at least a three $\log_{10}$, preferably at least a four $\log_{10}$ relative to a non-irradiated pancreas gland. The PPV infectiousness of an enzyme preparation may be further reduced by subsequent, orthogonal viral inactivation steps performed following irradiation.

The methods can also include the step of, following the irradiation step, testing for the presence or amount of one or more microorganisms (*e.g.*, viruses, bacteria, or protozoa) in the tissue or enzyme preparation derived from the tissue. Methods for determining whether a sample contains a microorganism are known in the art and include, for example, plaque-assays or colony formation tests. Effective sterilization can also be determined using conventional microbiological techniques, such as, for example, the inclusion of suitable biological indicators in a radiation batch or contacting the tissue with a culture medium, and incubating the medium to determine sterility of the tissue.

Viral infectiousness can be calculated by endpoint titration and subsequent calculation of the half tissue culture infectious dose ($TCID_{50}$). The virus titres calculated in this manner can be given as $\log_{10} TCID_{50}$ per ml with confidence intervals of 95%.

The reduction in viral contamination is given in accordance with USP-NF general chapter <1050>, as a logarithmic reduction factor which is the difference in virus titre between a control sample and the sample derived from an e-beam irradiated tissue upon isolation. For example, a 3 $\log_{10}$ reduction may indicate a reduction in viral load by a factor of 1,000 and a 4 $\log_{10}$ reduction may indicate a reduction in viral load by a factor of 10,000.

The methods of manufacturing the pancreatin enzyme preparation may allow for the reduction of viral and/or microbial contamination of the enzyme preparation without a substantial reduction in its enzymatic activity.

Enzymatic activity of a pancreatin enzyme preparation isolated from an irradiated pancreas gland may be maintained. For example, , a biological activity of the enzyme preparation may be at least 50%, at least 60%, at least 70%, at least 80%, or preferably at least 90%, of the biological activity of a control preparation isolated from a non-irradiated pancreas gland. The biological activity may be enzymatic activity, such as protease activity, amylase activity, or, preferably, lipase activity.

Following irradiation, the intact mammalian pancreatic tissue is further processed to provide the enzyme composition by isolating one or more enzymes and/or proenzymes Various methods for isolating enzymes from tissue samples are known. For example, US 4,623,624 provides methods for isolating pancreatin by autolysis of an aqueous isopropanol-containing tissue slurry.

The irradiated tissue may be subjected to autolysis and/or hydrolysis to convert proenzymes to their active form. For example, the irradiated tissue may be combined with a hydrolysis starter to initiate autolysis. The autolysis and/or hydrolysis may be carried out at ambient temperature. The reaction mixture may be filtered upon completion of the reaction; the filtrate may be collected; the enzymes present in the filtrate may be precipitated (e.g., with isopropanol); and the precipitate may be filtered, washed with isopropanol, and vacuum dried.

## C. COMPOSITIONS

In at least one aspect, the present invention includes pharmaceutical compositions comprising a pancreatin enzyme preparation as described herein. The compositions comprise one or more enzymes isolated from an irradiated intact mammalian pancreatic tissue. The composition may be a crude mixture containing one or more enzymes isolated from intact mammalian pancreatic tissue.

A pharmaceutical composition comprising a pancreatin enzyme preparation as described herein is provided, and may further optionally comprise one or more conventional pharmaceutically acceptable excipients such as those found in textbooks such as Remington's Pharmaceutical Sciences, 18th Ed. (Alfonso R. Gennaro, ed.; Mack Publishing Company, Easton, PA, 1990); Remington: the Science and Practice of Pharmacy 19th Ed. (Lippincott, Williams & Wilkins, 1995); Handbook of Pharmaceutical Excipients, 3rd Ed. (Arthur H. Kibbe, ed.; Amer. Pharmaceutical Assoc, 1999); the Pharmaceutical Codex: Principles and Practice of Pharmaceutics 12th Ed. (Walter Lund ed.; Pharmaceutical Press, London, 1994); The United States Pharmacopeia: The National Formulary (United States Pharmacopeial Convention); and Goodman and Gilman's: the Pharmacological Basis of Therapeutics (Louis S. Goodman and Lee E. Limbird, eds.; McGraw Hill, 1992).

Pharmaceutical compositions comprising a pancreatin enzyme preparation as described herein may be used to supplement digestive enzymes in the treatment and/or the prophylaxis of maldigestion due to chronic exocrine pancreatic insufficiency, which may be in patients suffering from cystic fibrosis, chronic pancreatitis or patients who have undergone upper gastrointestinal surgery. Pharmaceutical compositions or dosage forms as described herein may preferably be oral dosage forms which can in particular be administered to humans.

An oral dosage form containing an enzyme preparation may be in the form of, for example, capsules, granules, granulates, micropellets, microspheres, microtablets, pellets, pills, powders and/or tablets. For the purposes of this description, the prefix "micro" is used to describe an oral dosage form if the diameter of the oral dosage form or all of its dimensions (length, height, width) is equal to or below about 5 mm.

The oral dosage form may be a capsule. The capsule may comprise between about 2,000 and about 40,000 lipase units per capsule. The oral dosage form may be a capsule comprising 3,000, 6,000, 12,000, 24,000, or 36,000 lipase units per capsule. The oral dosage form may be a capsule comprising 3,000, 5,000, 10,000, 15,000, 20,000, 25,000, or 40,000 lipase units per capsule. The oral dosage form may be a capsule comprising 2,600, 4,200, 10,500, 16,800, or 21,000 lipase units per capsule. The oral dosage form may be a capsule comprising 4,000, 13,800, 20,700, or 23,000 lipase units per capsule. The oral dosage form may be a capsule comprising 4,000, 8,000, or 16,000 lipase units per capsule.The oral dosage form may be a capsule comprising 4,000, 8,000, or 16,000 lipase units per capsule. The oral dosage form may be a capsule comprising 3,000, 4,000, 6,000, or 8,000 lipase units per capsule. Dosage strength may be expressed in a variety of ways, including in USP units, Ph.Eur. units, or BP units.

The oral dosage form may be a tablet comprising 10,440 or 20,880 lipase units per tablet.

Various pharmaceutical compositions and dosage forms containing pancreatin are known, such as delayed release and immediate release compositions. For example, US 9,198,871 provides delayed release pancreatin compositions.

The oral dosage form may be a pancreatin micropellet or pancreatin microsphere. The pancreatin micropellet or pancreatin microsphere may be coated with, for example, an enteric coating. The pancreatin micropellet or pancreatin microsphere - independent of any such coating - may comprise between about 10% and about 95% by weight of pancreatin, between about 5% and about 90% by weight of at least one pharmaceutically acceptable binding agent, and between 0% and about 10% by weight of at least one pharmaceutically acceptable excipient. More specifically, the pancreatin micropellet or pancreatin microsphere may comprise between about 70% and about 90% by weight of pancreatin, between about 10% and about 30% by weight of at least one pharmaceutically acceptable binding agent, and between 0% and about 5% by weight of at least one pharmaceutically acceptable excipient. The pancreatin micropellet or pancreatin microsphere may comprise between about 70% and about 90% by weight pancreatin and between about 10% and about 30% by weight of at least one pharmaceutically acceptable binding agent. The pancreatin micropellet or pancreatin microsphere may be approximately spherical and has a diameter between about 0.5 mm and about 2.0 mm.The pancreatin micropellet or pancreatin microsphere may have a first dimension between about 0.5 mm and about 2.0 mm and a second dimension between about 0.5 mm and about 2.0 mm. The pancreatin micropellet or pancreatin microsphere may have a first dimension between about 0.8 mm and about 1.0 mm and a second dimension between about 0.5 mm and about 2.0 mm.

Examples of pharmaceutically acceptable binding agents may include polyethylene glycol 1500, polyethylene glycol 2000, polyethylene glycol 3000, polyethylene glycol 4000, polyethylene glycol 6000, polyethylene glycol 8000, polyethylene glycol 10000, hydroxypropyl methylcellulose, polyoxyethylene, copolymers of polyoxyethylen-polyoxypropylen and mixtures of said organic polymers. Polyethylene glycol 4000 may be a preferred pharmaceutically acceptable binding agent.

Examples of suitable pharmaceutically acceptable excipients may include gliding agents like magnesium stearate or calcium stearate, stearic acid, talcum and/or starch; fillers like calcium phosphate, corn starch, dextrans, dextrin, hydrated silicon dioxide, microcrystalline cellulose, kaolin, lactose, mannitol, polyvinyl pyrrolidone, precipitated calcium carbonate, sorbitol and/or talcum; disintegrating agents like Aerosil (silicic acid), alginic acid, amylose, calcium alginate, calcium carbonate, formaldehyde gelatin, pectic carbonate, sago starch, sodium bicarbonate and/or starch; and/or moisturizers like glycerol and/or starch. For the purposes of the present disclosure, synthetic oils and monomeric phthalic acid esters may not to be regarded as suitable pharmaceutically acceptable excipients. The pancreatin micropellets or pancreatin microspheres may contain no pharmaceutically acceptable excipients, but can optionally contain a greater amount of pancreatin.

An oral dosage form, such as an enteric coated oral dosage form, of pancreatin may be provided for the manufacture of a medicament for the treatment of medical conditions such as digestive disorders, pancreatic exocrine insufficiency, pancreatitis, cystic fibrosis, diabetes type I and/or diabetes type II.

The pharmaceutical composition may be a controlled release pharmaceutical composition. For example, a controlled release pharmaceutical composition can be obtained by applying an enteric coating to an oral dosage form. The enteric coating may comprise a film-forming agent, a plasticizer, and, optionally, an anti-sticking agent.

Suitable film-forming agents may include agar, carbomer homopolymer and copolymers (*i.e.,* high molecular weight, crosslinked, acrylic acid-based polymers), carboxymethyl cellulose, carboxymethylethyl cellulose, carrageen, cellulose acetate phthalate, cellulose acetate succinate, cellulose acetate trimelliate, chitin, corn protein extract, ethyl cellulose, gum arabic, hydroxypropyl cellulose, hydroxypropylmethyl acetate succinate, hydroxypropyl methylcellulose acetate succinate, hydroxypropyl methylcellulose phthalate, methacrylic acid-ethyl methacrylate-copolymer, methyl cellulose, pectin, polyvinyl acetate phthalate, polivinyl alcohol, shellac, sodium alginate, starch acetate phthalate and/or styrene/maleic acid copolymer or mixtures of said film-forming polymers. Cellulose acetate phthalate, hydroxypropyl methylcellulose acetate succinate and/or methacrylic acid-ethyl methacrylate-copolymer are the preferred film-forming agents. Most preferred is hydroxypropyl methylcellulose phthalate, such as HP 55 or HPMCP HP-50. Synthetic oils are not to be regarded as preferred film-forming agents.

The plasticizer(s) may be present in an amount greater than about 1.5%, and typically in an amount between about 2% and about 20% by weight, relative to the film-forming agent. The plasticizer may contain saturated linear monohydric alcohols having 12 to 30 carbon atoms. More specifically, acceptable plasticizers include lauryl alcohol, tridecyl alcohol, myristyl alcohol, pentadecyl alcohol, cetyl alcohol, heptadecyl alcohol, stearyl alcohol, nonadecyl alcohol, arachic alcohol, behenyl alcohol, carnaubyl alcohol, ceryl alcohol, corianyl alcohol, melissyl alcohol, acetyl tributyl citrate, dibutyl sebacate, fatty acid esters of glycerol, glycerol, polyethylene glycol, propyleneglycol, sorbitan fatty acids, triacetin, triethyl citrate and mixtures of said plasticizers. Preferred plasticizers are cetyl alcohol, stearyl alcohol, triethyl citrate and mixtures thereof. When cetyl alcohol is used as a single plasticizer, it may be present in an amount of greater than about 1.5%, typically in an amount of about 2% to about 15%, preferably about 2% to about 10%, by weight relative to the film-forming agent. When triethyl citrate is used as a single plasticizer, it may be present in an amount between about 5% and about 20%, preferably between about 12% and about 15%, by weight relative to the film-forming agent. Synthetic oils and monomeric phthalic acid esters are not to be regarded as suitable plasticizers.

The plasticizer may be comprised of cetyl alcohol and triethyl citrate which collectively present in an amount of greater than about 3%, typically in an amount of about 4% to about 20%, in particular between about 6% and about 15%, more particularly between about 7% and about 10%, by weight in relation to the film-forming agent. The weight to weight ratio of cetyl alcohol to triethyl citrate when both are present may be from about 0.05:1 to about 1:1, for example 0.1:1, 0.2:1, 0.3:1, 0.4:1, 0.5:1, 0.6:1, 0.7:1, 0.8:1 or 0.9:1. In particular, the ratio of cetyl alcohol to triethyl citrate may be from about 0.25:1 to about 0.5:1, preferably from about 0.3:1 to about 0.45:1, more preferably from about 0.35:1 to about 0.4:1, and even more preferably from about 0.38:1 to about 0.4:1 (w/w).

The enteric coating optionally may comprise an anti-sticking agent. Suitable anti-sticking agents may include dimethicone and castor oil. Dimethicone, in particular dimethicone 1000, is the preferred anti-sticking agent. The amount of anti-sticking agent (if present) in the enteric coating is between about 1.5% and about 3% by weight relative to the film-forming agent. Synthetic oils are not to be regarded as preferred anti-sticking agents.

The enteric coating may comprise between about 5% and about 30% by weight, more preferably between about 7% and about 20% by weight, yet more preferably between about 10% and about 15% by weight of the total composition of the enteric coated oral dosage form or controlled release pharmaceutical composition. The enteric coating may comprise between about 20% and about 30% by weight, more preferably between about 22% and about 26% by weight, yet more preferably between about 22.5% and about 25% by weight of the total composition of the enteric coated oral dosage form or controlled release pharmaceutical composition.

The pharmaceutical composition may be a controlled release capsule for oral administration. The capsule may contain enteric-coated pellets comprising lipase, protease, and amylase. The enteric-coated pellets may have a first dimension between about 0.5 mm and about 2.0 mm and, optionally, a second dimension between about 0.5 mm and about 2.0 mm. For example, the enteric-coated pellets may be approximately spherical and have a diameter from about 0.71 to about 1.60 mm. As another example, the enteric-coated pellets may be strand-like and have a diameter from about 0.5 mm and about 2.0 mm and a length from about 0.5 mm and about 2.0 mm. The composition may further include inactive ingredients described herein, such as cetyl alcohol, dimethicone, hypromellose phthalate, polyethylene glycol, and triethyl citrate.

The pharmaceutical composition may be an immediate release pharmaceutical composition. For example, the immediate release pharmaceutical composition may lack an enteric coating.

## D. METHODS OF USE

In at least one aspect, the present invention includes pancreatin or a pharmaceutical composition comprising the pancreatin as disclosed herein for use in treating exocrine pancreatic insufficiency, wherein in a subject, in particular a human subject, in need of such treatment, wherein, the exocrine pancreatic insufficiency may be due to cystic fibrosis, chronic pancreatitis, pancreatectomy, or other conditions.

As related to the methods and uses mentioned above, the enzyme preparation comprises pancreatin. A suitable dosage of pancreatin may be based on lipase units. The Cystic Fibrosis Foundation (CFF) has published Consensus Guidelines that contain recommended total daily doses of lipase units.

From 2,000 to 4,000 lipase units, preferably 3,000 lipase units, per 120 mL of formula or per breast-feeding may be administered to an infant up to 12 months of age.

From 1,000 to 2,500 lipase units per kg of body weight per meal may be administered to an individual from one (1) to four (4) years of age. From 500 to 2,500 lipase units per kg of body weight per meal may be administered to an individual at least four (4) years of age.

The maximum daily dosage may not exceed 10,000 lipase units per kg of body weight. The maximum daily dosage may not exceed 4,000 lipase units per g of fat ingested.

The enzyme preparation or the pharmaceutical composition containing the enzyme preparation may be administered immediately prior to a meal. The enzyme preparation or the pharmaceutical composition containing the enzyme preparation may be administered during a meal or a snack.

The enzyme preparation comprises one or more enzymes isolated from the electron beam irradiated intact mammalian pancreatic tissue. The enzyme preparation may be in a crude form.

The enzyme preparations, compositions, methods, and uses described herein will be better understood by reference to the following exemplary embodiments and examples.

## E. EXEMPLARY EMBODIMENTS

One aspect of the present invention includes a pancreatin produced by a method comprising the steps of: (a) subjecting intact mammalian pancreatic tissue to electron beam radiation to produce irradiated pancreatic tissue, wherein the electron beam radiation is sufficient to produce at least a three $\log_{10}$ reduction in viral load of a model virus compared to a control sample; and (b) isolating pancreatin from the irradiated pancreatic tissue. The isolating step may comprise initiating hydrolysis or autolysis of the pancreatic tissue. The isolating step may comprise mixing the irradiated pancreatic tissue with water. Step (b) may comprise activating a proenzyme from the irradiated pancreatic tissue. The reduction in microbial and/or viral load may be at least a three $\log_{10}$, preferably at least a four $\log_{10}$, reduction relative to a control sample obtained from non-irradiated tissue. For example, the reduction in microbial and/or viral load may be at least a

three $\log_{10}$, preferably at least a four $\log_{10}$, reduction in PPV viral load relative to a control sample obtained from non-irradiated tissue. In certain embodiments, a biological activity of the enzyme preparation obtained in step (b) corresponds to at least 50%, preferably at least 90%, of the biological activity of a control enzyme preparation obtained from non-irradiated pancreatic tissue. In certain embodiments, the biological activity is lipase activity. The biological activity may be protease activity or amylase activity. The method may further comprise one or more additional steps that provide an additional reduction in microbial and/or viral load.

According to the invention, prior to enzyme isolation, the mammalian tissue has been subjected electron bean irradiation sufficient to produce at least a three $\log_{10}$, preferably at least a four $\log_{10}$, reduction in viral load. For example, the treatment may be sufficient to produce at least a three $\log_{10}$, preferably at least a four $\log_{10}$, reduction in PPV viral load relative to an untreated control sample. The mammalian tissue may be a porcine pancreatic gland. The porcine pancreatic gland may be flaked. The porcine pancreatic gland may be in a frozen block. The porcine pancreatic gland may be a whole gland or portion thereof, such as one or more lobes. The one or more enzymes comprise pancreatin. The treatment comprises electron beam radiation and may be at a dose from about 5 to about 50 kGy, preferably from about 10 to about 40 kGy. In certain embodiments, a biological activity of the enzyme preparation corresponds to at least 50%, preferably at least 90%, of the biological activity of a control enzyme preparation obtained from a control enzyme preparation. In certain embodiments, the biological activity is lipase activity. The biological activity may be protease activity or amylase activity. The reduction in viral load is an orthogonal reduction.

The present disclosure includes a pharmaceutical composition comprising the pancreatin isolated from intact mammalian pancreatic tissue that has been subjected to electron beam irradiation to reduce risk of viral and microbial infectivity, wherein the composition may have a biological activity that corresponds to at least 50%, preferably at least 90%, of the biological activity of a control composition. The control composition may comprise an untreated sample of mammalian tissue corresponding to the mammalian tissue that may have been subjected to a treatment to reduce risk of viral and microbial infectivity. The electron beam radiation may have a dose from about 5 to about 50 kGy, preferably from about 10 to about 40 kGy.

Another aspect of the present invention includes a method for producing a pancreatin product comprising the steps of: (a) subjecting intact mammalian pancreatic tissue to electron beam radiation to produce irradiated pancreatic tissue; and (b) isolating pancreatin from the irradiated pancreatic tissue. In certain embodiments, a biological activity of the pancreatin product obtained in step (b) corresponds to at least 50%, preferably at least 90%, of the biological activity of a control pancreatin product. The control pancreatin product may be obtained from non-irradiated pancreatic tissue. In certain embodiments, the electron beam radiation is sufficient to produce at least a three $\log_{10}$, preferably at least a four $\log_{10}$, reduction in viral load. For example, the treatment is sufficient to produce a reduction in viral load is at least a three $\log_{10}$, preferably at least a four $\log_{10}$, reduction in PPV viral load relative to a non-irradiated control sample. The electron beam radiation may have a dosage from about 5 to about 50 kGy, preferably from about 10 to about 40 kGy. In certain embodiments, the biological activity is lipase activity. The biological activity may be protease activity or amylase activity.

The present disclosure includes a method for digesting a protein that may comprise the steps of: (a) providing an enzyme or proenzyme preparation isolated from an electron beam irradiated animal tissue; and (b) contacting the protein with the enzyme or proenzyme preparation under conditions sufficient to digest the protein. In certain embodiments, the step of contacting occurs *in vivo*. The step of contacting may occur *in vitro*. The animal tissue may be porcine pancreas. The digested protein may be used to prepare a protein hydrolysate product. The enzyme or proenzyme preparation derived from an electron beam irradiated animal tissue may exhibit at least a three $\log_{10}$, preferably at least a four $\log_{10}$, reduction in viral load compared to a control enzyme or proenzyme preparation derived from non-irradiated animal tissue. The enzyme or proenzyme preparation derived from an electron beam irradiated animal tissue may exhibit a biological activity corresponding to at least 50%, preferably at least 90%, of the biological activity of a control enzyme or proenzyme preparation derived from non-irradiated animal tissue.

Another aspect of the present invention includes a pancreatin isolated from electron beam irradiated intact mammalian pancreatic tissue. The pancreatic tissue may comprise a porcine pancreatic gland. The porcine pancreatic gland may be frozen and mechanically processed into flakes or blocks prior to irradiation. Thus, the pancreatic tissue subject to electron beam irradiation may be flaked frozen mammalian pancreatic tissue or a frozen block of mammalian pancreatic tissue. The porcine pancreatic gland may be a whole gland or portion thereof, such as one or more lobes. The one or more enzymes may comprise pancreatin. The enzyme preparation may exhibit at least a three $\log_{10}$, preferably at least a four $\log_{10}$, reduction in viral load compared to a control enzyme preparation. For example, the enzyme preparation may exhibit at least a three $\log_{10}$, preferably at least a four $\log_{10}$, reduction in PPV viral load relative to a control enzyme preparation. In certain embodiments, a biological activity of the enzyme preparation corresponds to at least 50%, preferably at least 90%, of the biological activity of a control enzyme preparation. In certain embodiments, the biological activity is lipase activity. The biological activity may be protease activity or amylase activity. The control enzyme preparation may be obtained from non-irradiated tissue. The reduction in viral load may be an orthogonal reduction.

Another aspect of the present invention includes a pancreatin or a pharmaceutical composition comprising pancreatin as disclosed herein for use in treating exocrine pancreatic insufficiency. In certain embodiments, the exocrine pancreatic insufficiency is due to cystic fibrosis or chronic pancreatitis.

## F. EXAMPLES

### Example 1. E-beam irradiation of porcine parvovirus (PPV), pancreatin API, and porcine pancreas

*Materials and Methods.*

*Vialed Porcine Parvovirus in Cell Culture Fluid.* Because the porcine gland tissue itself may have some inactivating effects on viruses, initially, the virus samples used for these studies were prepared from infected cell cultures. Porcine Parvovirus (PPV), Strain NADL-2 (ATCC® VR-742™) and Pig Testis (ST) cells (ATCC® CRL-1746™) were purchased from American Type Culture Collection (ATCC). PPV was propagated, cultured and maintained according to ATCC recommendations. PPV was propagated to an approximate titer of $10^8$ virus Infectious Units (IU)/ml. Virus was harvested from lysed cells in cell culture media consisting of Minimum Essential Medium (MEM), 10% Fetal Bovine Serum (FBS), 2 mM Glutamine, 100 units /ml penicillin, 100 mg/ml streptomycin.

PPV was packaged in vials and then double bagged prior to shipment. The vials were Nalgene Cryogenic Vials (i.e., polypropylene with externally threaded high density polyethylene (HDPE) closure with a leak-proof sealing ring having a length of 1.87 in; a diameter of 0.5 in.; a capacity of 2.0 ml; and fill volume of 1.0 ml. Each individual vial was placed in a Food Saver bag (polyethylene with an outer layer of nylon) and vacuum sealed. The bags were trimmed to just fit the vial. Four individually sealed vials were then placed inside another Food Saver bag (approximately 11X14") and vacuum sealed.

*Frozen Flaked Porcine Pancreas Glands.* Pancreas glands obtained from butcher hog (Animal Technologies, Tyler, TX) were kept on dry ice.

Frozen flaked porcine pancreas glands were placed in a 12" × 12" × 1 ¾" container (clear polypropylene), which was vacuumed sealed inside a clear 3 mil poly/nylon bag. The bag was heat sealed. The pancreas glands were held on dry-ice to ensure temperatures below -20°C. The sample weight of the frozen flaked porcine pancreas gland was 1.05 kg plus a packaging weight of 270 grams (0.27 kg). The lid weighed 100g. The surface density was 1.3 g/cm$^2$. Two packages were made for each radiation dose including control without radiation, one intact package was used for isolation of the enzyme preparation after transit back to the site where the isolation was conducted with the other package being a reserve.

*Pancreatin (API).* Two types of pancreatin API were used: Pancreatin N and Pancreatin S. Both API were held at ambient conditions. Pancreatin N (Material # 1030828, Abbott Laboratories) is a light yellow/gray to off-white powder. Pancreatin N originated from butcher hog pancreas. Pancreatin S (Material #1030829, Abbott Laboratories) is a light yellow/gray to off-white powder. Pancreatin S originated from sow pancreas.

Pancreatin API was placed in a 2 ½" × 3 ½" × 1 ⅝" container (clear polypropylene), which was vacuumed sealed inside a clear 3 mil poly/nylon bag. The bag was heat sealed. The sample weight of the pancreatin API was 80 grams plus a packaging weight of 26 grams. The surface density was 1.4 g/cm$^2$. Samples of Pancreatin N were exposed to electron beam radiation at the indicated dose. The control was not exposed to electron beam radiation. Pancreatin N was assayed for activity after transit back to the experimental site. Pancreatin N was derived from butcher hog. All Pancreatin N packages arrived intact from the irradiation site.

*E-beam irradiation.*

The source of the electron beam was an Industrial Materials Processing Electron Linear Accelerator (IMPELA®; Iotron Industries, Inc. Columbia City, IN), 10 MeV, 80 cm scan width.

The samples of packaged frozen flaked porcine pancreas and porcine parvovirus were held below -20°C by placing on dry ice contained in an aluminum tray. Dosimeters were placed on the upper surface of each sample. The samples of packaged pancreatin API maintained at ambient temperature were placed on an expanded polystyrene foam sheet, dosimeters were placed on the upper surface. The packages were sent on a conveyor belt under the E Beam scan horn. After one pass of radiation, the dosimeters were retrieved. The packages were sent below the radiation horn for a second pass after inverting the package (upper side is now facing down) and a new dosimeter was placed on the upper surface. After the second round of radiation, the packages and dosimeters were retrieved. The frozen flaked porcine pancreas and porcine parvovirus packages were retrieved and placed on dry ice for shipment. The packages of pancreatin API were retrieved and held at ambient temperature for shipment. The control packages of frozen flaked pancreas and porcine parvovirus placed on dry ice were prepared for re-shipment on dry ice without undergoing radiation. The control packages of pancreatin API held at ambient temperature were prepared for re-shipment at ambient without undergoing radiation. The electron beam dosage was calculated from the dosimeter exposure using a calibration curve.

*Viral Infectivity Testing.*

Viral infectivity was determined by titration of ten-fold dilutions in 96 well microplates using appropriate controls for triplicate samples at each energy dose. The virus titers were calculated using the Reed and Muench method described in Reed, L.J.; Muench, H. (1938). "A simple method of estimating fifty percent endpoints." The American Journal of Hygiene 27: 493-497 and expressed as a 50% $TCID_{50}$ per mL.

*Pancreatin Isolation and Testing.*

The method used for isolation of pancreatin is substantially similar to that described in US 4,623,624 that comprises hydrolysis and/or autolysis, followed by fiber sieving, precipitation of enzymes, separation of precipitate by filtration and/or centrifugation, washing of the cake, drying and particle size reduction. Hydrolysis was performed using one package corresponding to each radiation dosage including the unirradiated control. A package without damage to the packaging (such as large cracks) was selected for each isolation experiment. Due to the care taken during transit back to the experimental site, all packages arrived intact and one package was selected at random for each radiation dosage including control for isolation. Pancreatin from an earlier isolation was used as a protease source to start the hydrolysis. Calcium hydroxide was used to supply calcium ions needed for activation of the proteases. Sodium bicarbonate is used as a buffering agent for the hydrolysis. Simethicone was used as an antifoam agent. The hydrolysis of the pancreas was performed close to ambient temperature. The completion of hydrolysis was checked by centrifugation of the hydrolyzed mixture after addition of isopropanol as described below. After completion of hydrolysis additional isopropanol was added to reduce the hydrolysis rate, the mixture was cooled, the mixture was stirred and fibers were separated using a 0.425 inch mesh. The fibers were washed with isopropanol and compressed to expel the liquid that is held. The wash was combined with the filtrate obtained earlier. Additional isopropanol was added to the filtrate to cause precipitation of the enzymes. The suspension was filtered through filter cloth with 15 micron openings and washed with increasing concentrations of isopropanol with isopropanol absolute as the final wash. The cake was dried on the filter cloth under nitrogen flow while pulling vacuum below the filter cloth till the cake appeared dry visually (color of cake turns lighter after removal of isopropanol and water). The cake was peeled away from the filter cloth and dried under vacuum and nitrogen flow at temperature below about 50°C to a water content by Karl Fischer of 3.5% or lower. The yield of dry pancreatin is 80 to 100 g from 1 Kg of frozen flaked porcine pancreas from butcher hog.

*Centrifugation Test for Completion of Hydrolysis.* Sample three scoops of about 10 ml from the hydrolysis vessel and pass through 0.425 mm sieve, collect filtrate into plastic beaker (scrape filtrate into plastic beaker also), discard the fibers that are retained on the mesh. Using a pipette, add 10 g solution from reactor to 50 mL centrifuge tube, add 5.5mL IPA 85%, stir for 1 minute with spatula. Add 20 mL IPA 85% to filtrate in 50 mL centrifuge tube. Stir for one minute with spatula. Centrifuge suspension at approximately 90X g for two minutes.

The first sample may be taken two hours after start of hydrolysis or when color is changing from pink to brownish and suspension becomes thinner (about 2 hours).

The next sample is taken when color is brown without pink shade, at this point sediment is expected to be about 25%, the sediment will be less firm and stragglers may be present in clear layer above. After this, sampling may be done at every half an hour intervals as possible.

Hydrolysis stopped if two subsequent measurements show less than 20% of sediments. At this point sediment will be firm and without stragglers in clear layer above.

Two-sided electron beam irradiation of vialed PPV was performed in triplicate. Data for viral load reduction and log kill of PPV exposed to electron beam radiation are shown in Table 1:

| Dosage (KGy) | 0 | 9.5 | 19.25 | 38.45 | 0 | 9.5 | 19.25 | 38.45 |
|---|---|---|---|---|---|---|---|---|
| | Viral Load | Viral Load | Viral Load | Viral Load | Log Kill | Log Kill | Log Kill | Log Kill |
| | Virus titer/mL | Virus titer/mL | Virus titer/mL | Virus titer/mL | | | | |
| Test A | 1.50E+08 | 2.50E+06 | 1.50E+05 | | 0.00E+00 | 1.78E+00 | 3.00E+00 | |
| Test B | 1.50E+08 | 6.34E+06 | 6.34E+04 | | 0 | 1.37E+00 | 3.37E+00 | |
| Test C | 4.00E+08 | 1.26E+06 | 4.00E+05 | 1.50E+02 | 0 | 2.50E+00 | 3.00E+00 | 6.43E+00 |
| **Mean** | | | | | **0** | **1.88E+00** | **3.12E+00** | **6.43E+00** |

E-beam dosage was determined by estimating the dose absorbed by each sample from the surface dose as indicated by the dosimeter affixed to the sample container.

Table 1 shows that exposure of PPV to about 40 kGy provides about a 6.5 $\log_{10}$ kill, while exposure to about 20 kGy provides about a 3 $\log_{10}$ kill. Based on these data, it is expected that exposure to about 30 kGy will provide at least a 4 $\log_{10}$ kill.

Exposure of PPV to about 60 kGy, about 80 kGy, or about 100 kGy resulted in final viral titers below the limit of detection of the assay.

Pancreatin was tested for free protease, amylase, and lipase activity as described in USP by validated methods. Pancreatin was tested for total protease as described in European Pharmacopoeia (EP) by validated methods.

Two-sided electron beam irradiation of Pancreatin N was performed. All the Pancreatin N containers were received intact at the experimental site and were used for assay. The Data for enzymatic activity of pancreatin API exposed to electron beam radiation are shown in Table 2:

| E Beam Dose | Lipase Activity | Amylase Activity | Total Protease Activity | Free Protease Activity |
| --- | --- | --- | --- | --- |
| KGy | USP Units/gram | USP Units/gram | USP Units/gram | USP Units/gram |
| Control (0 kGy) | 89106 | 564390 | 383192 | 314093 |
| 18.6 kGy | 63328 | 398752 | 321567 | 266540 |
| 37.45 kGy | 58631 | 386628 | 302341 | 244531 |
| 56.5 kGy | 47755 | 282378 | 272499 | 232087 |
| 76.35 kGy | 40583 | 272760 | 259117 | 214914 |
| 99.4 kGy | 37799 | 286652 | 247005 | 196356 |

Table 2 shows that exposure of pancreatin API to about 20 kGy provides about a 30% loss in lipase activity, exposure of pancreatin API to about 40 kGy provides about a 35% loss in lipase activity, exposure of pancreatin API to about 60 kGy provides about a 46% loss in lipase activity, exposure of pancreatin API to about 80 kGy provides about a 54% loss in lipase activity, and exposure of pancreatin API to about 100 kGy provides about a 58% loss in lipase activity. Based on these data, it is expected that exposure of pancreatin API to about 30 kGy will provide at least a 30% loss in lipase activity.

Two-sided electron beam irradiation of butcher hog pancreas was performed. Data for enzymatic activity of pancreatin derived from frozen flaked porcine pancreas gland exposed to electron beam radiation are shown in Table 3:

| E Beam Dose | Lipase Activity | Amylase Activity | Total Protease Activity | Free Protease Activity |
| --- | --- | --- | --- | --- |
| KGy | USP Units/gram | USP Units/gram | USP Units/gram | USP Units/gram |
| Control (0 kGy) | 74986 | 423487 | 243421 | 151365 |
| 18.75 kGy | 74741 | 449569 | 337594 | 128749 |
| 35.5 kGy | 65348 | 349816 | 360407 | 128506 |
| 56.55 kGy | 57976 | 398281 | 268297 | 119727 |
| 77.4 kGy | 34806 | 251691 | 175994 | 109158 |
| 100.4 kGy | 36362 | 276118 | 206217 | 100342 |

Table 3 shows that exposure of frozen flaked porcine pancreas glands to about 20 kGy provides about a 1% loss in lipase activity, exposure of frozen flaked porcine pancreas glands to about 40 kGy provides about a 13% loss in lipase activity, exposure of frozen flaked porcine pancreas glands to about 60 kGy provides about a 23% loss in lipase activity, exposure of frozen flaked porcine pancreas glands to about 80 kGy provides about a 54% loss in lipase activity, and exposure of frozen flaked porcine pancreas glands to about 100 kGy provides about a 52% loss in lipase activity. Based on these data, it is expected that exposure of frozen flaked porcine pancreas glands to about 30 kGy will provide about a 10% loss in lipase activity.

As shown in Tables 2 and 3, electron beam irradiation of the pancreatin API produces more loss of enzyme activity as compared to electron beam irradiation of the pancreatic tissue prior to isolation. Without wishing to be bound by theory, the resistance of the intact tissue to electron beam irradiation may be due to the conformation of the enzyme (*e.g.,* as a proenzyme) in the source tissue and/or co-factors in the source tissue providing structural protection.

**Example 2. E-beam irradiation of whole porcine pancreas glands**

A further study was performed using whole porcine pancreas. Approximately 3.6 kg thawed porcine pancreas was packed into wax lined cardboard boxes that were approximately 10 × 15 × 1.5 inches thick. The boxes were frozen to -20°C and held until use for e-beam irradiation. Boxes were shipped for e-beam irradiation by frozen truck (-20°C). Boxes were removed from the frozen truck and then exposed to double-sided electron beam irradiation - unirradiated boxes served as a control. Five (5) boxes were used for each nominal radiation dose: 0, 15, 20, and 25 kGy and shipped back for evaluations by frozen truck (-20°C), including the unirradiated boxes, and then held at -20°C.

Frozen pancreas samples were selected from each box at random locations within the box for subsequent isolation of pancreatin. Isolation was performed as described herein. Pancreatin isolated from electron beam irradiated whole pancreas was then tested for enzyme activity according to a colorimetric assay.

Pancreatin samples (API) were tested using colorimetric kinetic analysis by a microplate reader with the use of substrates structurally similar to those used in USP 39 <Pancrelipase>. The enzyme activities were determined by measuring the production rate of the product relative to the pancrelipase reference standard. Analytical comparability has been demonstrated between the filed USP monograph methods and the alternate microplate reader methods.

The sampling, isolating and assaying procedure was repeated four times for each dose to obtain a total of five measurements for each dose. Mean values of five measurements are reported in Table 4.

Table 4: API Enzyme Activity after Irradiation of Whole Pancreas Glands

| E Beam Dose* | Mean Lipase Activity | Mean Amylase Activity | Mean Free Protease Activity | Mean Total Protease Activity |
|---|---|---|---|---|
| KGy | USP Units/mg | USP Units/mg | USP Units/mg | USP Units/mg |
| Control (0 kGy) | 104 | 456 | 197 | 344 |
| 14.9 kGy | 99 | 435 | 201 | 291 |
| 19.9 kGy | 106 | 411 | 200 | 316 |
| 24.9 kGy | 104 | 395 | 183 | 301 |
| * (minimum dose + maximum dose)/2 | | | | |

Table 4 shows that pancreatin isolated from a whole pancreas gland exposed to a dose of up to about 25 kGy electron beam irradiation has the same or substantially the same lipase activity as pancreatin isolated from a unirradiated control.

**Example 3. Low dose E-beam irradiation of intact porcine pancreas tissue**

Further studies have been performed by spiking porcine pancreas tissue with live virus and then subjecting the virus-spiked tissue to electron beam radiation at lower dose (about 12.3 kGy) to enable virus recovery and assessment. This additional work was done to demonstrate effective inactivation of several related viruses at a low dose which would enable the effective enumeration of the impact of E-beam irradiation.

Selected viruses were deliberately spiked onto the tissue sample and the degree of virus clearance was evaluated by comparison of the amount of virus input to the amount of virus remaining after e-beam treatment. The viruses selected for this study are identified in Table 5.

Table 5: Viruses selected for viral clearance study

| Virus | Family | Genome | Envelope | Size (nm) | Physico-chemical resistance |
|---|---|---|---|---|---|
| Reovirus Type 3 (REO3) | Reo | RNA | No | 60-80 | Medium |
| Porcine Parvovirus (PPV) | Parvo | DNA | No | 18-24 | High |
| Feline Calicivirus (FCV) | Calici | RNA | No | 35-39 | Medium |

REO3 has a double-stranded RNA, segmented genome and belongs to the Reoviridae Family of viruses, which also includes rotavirus. Thus, REO 3 can serve as a model for rotavirus. FCV has been used as a model viruses for the validation of inactivation methods in blood products and, in particular, as a model for hepatitis E virus (HEV).

Porcine pancreas glands were sliced and ground. The tissue was then added to a petri dish, and spiked with the indicated virus. Standard virus stocks had certified titers of at least $1 \times 10^7$ pfu/mL. The spiked sample was incubated at room temperature for a minimum of 60 minutes (until the tissue returned to its original dryness). Following the incubation, additional porcine pancreas tissue was added to the dish. The dish was sealed and placed on dry ice for shipment to the e-beam facility. Each dish contained approximately 13 grams of tissue and the density of the tissue was similar to the density of a whole gland.

For each virus, spiked recovery samples (unshipped) and an unirradiated shipping control (shipped to ebeam facility but not irradiated) were included.

Electron beam irradiation was performed at the ebeam facility. After exposure to electron beam radiation was complete, the irradiated samples and the unirradiated shipping controls were shipped back for evaluation of viral load. Upon receipt, the samples were stored at ≤-60°C until testing.

For each sample, 50mL of culture media was added to a sterile bottle. The tissue was extracted by performing 3 rounds of soaking for approximately 5-10 minutes at room temperature followed by a 15-30 second vortex. The samples were then centrifuged at 3,000 RPM for 10 minutes. The supernatant from the extraction was used for viral testing.

Virus titers were determined by standard plaque assays. The indicator cell type for REO3, PPV, and FCV were Vero, PT-1, and CRFK, respectively. Virus titers and viral clearance factors were calculated according to standard procedures. The virus clearance factor (VCF) was calculated as follows:

$$VCF = \log_{10}\left[\frac{\text{Volume * titer before processing}}{\text{Volume * titer after processing}}\right]$$

Data for viral clearance produced by exposure of spiked tissue to electron beam radiation are shown in Table 6.

Table 6: Viral Clearance after Irradiation of Pancreas Glands Spiked with Virus

| Model Virus | Log Total Virus | | VCF | 95% Confidence Limit |
|---|---|---|---|---|
| | Before Processing | After Processing | | |
| REO3 | 6.5 | <2.4 | ≥4.1 | 0.08 |
| FCV | 6.7 | 4.9 | 1.8 | 0.05 |
| PPV | 5.8 | 3.6 | 2.2 | 0.29 |

These studies confirmed that sufficient inactivation of viruses, including feline calicivirus (FCV) and reovirus 3 (REO3), could be achieved with electron beam radiation of an intact tissue. Moreover, irradiation of a pancreas gland followed by isolation of an enzyme preparation (e.g., pancreatin API) from the irradiated gland showed similar results with respect to loss in enzyme activity relative to a non-irradiated control to those shown in Table 3 where flaked porcine pancreas was used.

The introduction of a decontamination step prior to processing the porcine pancreas provides effective control of known infectious agents both in terms of operator safety during the isolation of enzymes as well as patient safety. Thus, the use of an electron beam treatment that is effective to inactivate a wide spectrum of microbes and viruses, including difficult-to-inactivate viruses (*e.g.*, porcine parvo virus), with minimal loss in enzyme activity is advantageous over other methods.

As used herein, the electron beam radiation step can be considered an orthogonal viral inactivation step. The reduction in microbial and/or viral load obtained in the electron beam irradiated pancreatic tissue transfers to the pancreatin that is isolated from it in an additive manner with respect to the reduction obtained by other steps for microbial and/or viral reduction. Total $\log_{10}$ kill achieved by all the steps, including electron beam radiation, is the cumulative $\log_{10}$ kill achieved by all microbial and/or viral inactivation steps performed on the pancreatic tissue.

## Claims

1.    A pancreatin produced by a method comprising the steps of:

(a) subjecting intact mammalian pancreatic tissue to electron beam radiation to produce irradiated pancreatic tissue, wherein the electron beam radiation is sufficient to produce at least a three $\log_{10}$ reduction in viral load of a model virus compared to a control sample; and

(b) isolating pancreatin from the irradiated pancreatic tissue.

2. The pancreatin of claim 1, wherein the electron beam radiation is sufficient to produce at least a four $\log_{10}$ reduction in viral load of the model virus compared to the control sample.

3. The pancreatin of claim 1, wherein the model virus is porcine parvovirus (PPV).

4. The pancreatin of claim 1, wherein step (b) comprises initiating hydrolysis or autolysis of the irradiated pancreatic tissue or activating a proenzyme from the irradiated pancreatic tissue.

5. The pancreatin of any one of claims 1-4, wherein the electron beam radiation has a dosage from about 5 to about 50 kGy, preferably from about 10 to about 40 kGy.

6. A method for producing a pancreatin product comprising the steps of:

(a) subjecting intact mammalian pancreatic tissue to electron beam radiation to produce irradiated pancreatic tissue; and
(b) isolating pancreatin from the irradiated pancreatic tissue.

7. The method of claim 6, wherein a biological activity of the pancreatin product obtained in step (b) corresponds to at least 50%, preferably at least 90%, of the biological activity of a control pancreatin product, optionally wherein the biological activity is lipase activity.

8. The method of claim 6 or 7, wherein the electron beam radiation is sufficient to produce at least a three $\log_{10}$, preferably at least a four $\log_{10}$, reduction in viral load of a model virus compared to a control sample, wherein the model virus is preferably porcine parvovirus (PPV).

9. The method of any one of claims 6-8, wherein the electron beam radiation has a dosage from about 5 to about 50 kGy, preferably from about 10 to about 40 kGy.

10. A pharmaceutical composition comprising the pancreatin of any one of claims 1-5.

11. The pancreatin of any one of claims 1-5 or the pharmaceutical composition of claim 10 for use in treating exocrine pancreatic insufficiency, optionally wherein the exocrine pancreatic insufficiency is due to cystic fibrosis or chronic pancreatitis.

**Patentansprüche**

1. Pancreatin, hergestellt mit einem Verfahren, das die Schritte umfasst:

(a) Aussetzen von intaktem Pankreasgewebe eines Säugers einer Elektronenbestrahlung, um bestrahltes Pankreasgewebe herzustellen, wobei die Elektronenbestrahlung ausreichend ist, um eine Verringerung von mindestens drei $\log_{10}$ der Virenlast eines Modellvirus im Vergleich zu einer Kontrollprobe zu erreichen; und
(b) Isolieren von Pancreatin aus dem bestrahlten Pankreasgewebe.

2. Pancreatin nach Anspruch 1, wobei die Elektronenbestrahlung ausreichend ist, um eine Verringerung von mindestens vier $\log_{10}$ der Virenlast des Modellvirus im Vergleich zu der Kontrollprobe zu erreichen.

3. Pancreatin nach Anspruch 1, wobei der Modellvirus das porcine Parvovirus (PPV) ist.

4. Pancreatin nach Anspruch 1, wobei Schritt (b) das Initiieren einer Hydrolyse oder Autolyse des bestrahlten Pankreasgewebes oder das Aktivieren eines Proenzyms aus dem bestrahlten Pankreasgewebe umfasst.

5. Pancreatin nach einem der Ansprüche 1 bis 4, wobei die Elektronenbestrahlung eine Dosis von etwa 5 bis etwa 50 kGy aufweist, bevorzugt von etwa 10 bis etwa 40 kGy.

**6.** Verfahren zum Herstellen eines Pancreatinprodukts, das die Schritte umfasst:

(a) Aussetzen von intaktem Pankreasgewebe eines Säugers einer Elektronenbestrahlung, um bestrahltes Pankreasgewebe herzustellen; und
(b) Isolieren von Pancreatin aus dem bestrahlten Pankreasgewebe.

**7.** Verfahren nach Anspruch 6, wobei eine biologische Aktivität des in Schritt (b) erhaltenen Pancreatinprodukts mindestens 50%, bevorzugt mindestens 90% der biologischen Aktivität eines Kontroll-Pancreatinprodukts entspricht, gegebenenfalls wobei die biologische Aktivität Lipaseaktivität ist.

**8.** Verfahren nach Anspruch 6 oder 7, wobei die Elektronenbestrahlung ausreichend ist, um eine Verringerung von mindestens drei $\log_{10}$, bevorzugt mindestens vier $\log_{10}$ der Virenlast eines Modellvirus im Vergleich zu einer Kontrollprobe zu erreichen, wobei das Modellvirus bevorzugt porcines Parvovirus (PPV) ist.

**9.** Verfahren nach einem der Ansprüche 6 bis 8, wobei die Elektronenbestrahlung eine Dosis von etwa 5 bis etwa 50 kGy, bevorzugt von etwa 10 bis etwa 40 kGy aufweist.

**10.** Arzneimittel umfassend das Pancreatin nach einem der Ansprüche 1 bis 5.

**11.** Pancreatin nach einem der Ansprüche 1 bis 5 oder Arzneimittel nach Anspruch 10 zur Verwendung bei der Behandlung von exokriner Pankreasinsuffizienz, gegebenenfalls wobei die exokrine Pankreasinsuffizienz auf zystische Fibrose oder chronische Pankreatitis zurückzuführen ist.

**Revendications**

**1.** Pancréatine produite par une méthode comprenant les étapes de:

(a) soumission de tissu pancréatique intact de mammifère à une irradiation par faisceau d'électrons pour produire un tissu pancréatique irradié, dans laquelle l'irradiation par faisceau d'électrons est suffisante pour produire une réduction d'au moins trois $\log_{10}$ de la charge virale d'un virus modèle en comparaison avec un échantillon témoin; et
(b) isolation de pancréatine à partir du tissu pancréatique irradié.

**2.** Pancréatine selon la revendication 1, dans laquelle l'irradiation par faisceau d'électrons est suffisante pour produire une réduction d'au moins quatre $\log_{10}$ de la charge virale d'un virus modèle en comparaison avec un échantillon témoin.

**3.** Pancréatine selon la revendication 1, dans laquelle le virus modèle est le parvovirus porcin (PPV).

**4.** Pancréatine selon la revendication 1, dans laquelle l'étape (b) comprend l'initiation d'une hydrolyse ou autolyse du tissu pancréatique irradié ou l'activation d'une proenzyme provenant du tissu pancréatique irradié.

**5.** Pancréatine selon l'une quelconque des revendications 1 à 4, dans laquelle l'irradiation par faisceau d'électrons a une dose d'environ 5 à environ 50 kGy, de préférence d'environ 10 à environ 40 kGy.

**6.** Méthode pour produire un produit pancréatine, comprenant les étapes de:

(a) soumission de tissu pancréatique intact de mammifère à une irradiation par faisceau d'électrons pour produire un tissu pancréatique irradié; et
(b) isolation de pancréatine à partir du tissu pancréatique irradié.

**7.** Méthode selon la revendication 6, dans laquelle l'activité biologique du produit pancréatine obtenu dans l'étape (b) correspond à au moins 50 %, de préférence au moins 90 %, de l'activité biologique d'un produit pancréatine témoin, éventuellement dans laquelle l'activité biologique est l'activité de lipase.

**8.** Méthode selon la revendication 6 ou 7, dans laquelle l'irradiation par faisceau d'électrons est suffisante pour produire une réduction d'au moins trois $\log_{10}$, de préférence d'au moins quatre $\log_{10}$, de la charge virale d'un virus modèle en comparaison avec un échantillon témoin, dans laquelle le virus modèle est de préférence le parvovirus porcin (PPV).

9. Méthode selon l'une quelconque des revendications 6 à 8, dans laquelle l'irradiation par faisceau d'électrons a une dose d'environ 5 à environ 50 kGy, de préférence d'environ 10 à environ 40 kGy.

10. Composition pharmaceutique comprenant la pancréatine de l'une quelconque des revendications 1 à 5.

11. Pancréatine selon l'une quelconque des revendications 1 à 5 ou composition pharmaceutique selon la revendication 10, pour une utilisation dans le traitement d'une insuffisance pancréatique exocrine, éventuellement dans laquelle l'insuffisance pancréatique exocrine est due à une fibrose kystique ou à une pancréatite chronique.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 4623624 A
- US 20100119654 A
- WO 2003020324 A
- WO 2007014896 A
- US 20090233344 A
- US 9198871 B

**Non-patent literature cited in the description**

- Remington's Pharmaceutical Sciences. Mack Publishing Company, 1990
- Remington: the Science and Practice of Pharmacy. Lippincott, Williams & Wilkins, 1995
- Handbook of Pharmaceutical Excipients. Amer. Pharmaceutical Assoc, 1999
- Pharmaceutical Codex: Principles and Practice of Pharmaceutics. Pharmaceutical Press, 1994
- The United States Pharmacopeia: The National Formulary (United States Pharmacopeial Convention). **GOODMAN ; GILMAN'S.** Pharmacological Basis of Therapeutics. McGraw Hill, 1992
- **REED, L.J. ; MUENCH, H.** A simple method of estimating fifty percent endpoints. *The American Journal of Hygiene,* vol. 27, 493-497